# EUROPEAN PATENT APPLICATION

(11) **EP 3 095 397 A1**
(43) Date of publication of application: **23.11.2016**
(21) Application number: 14878838.3
(22) Date of filing: 09.10.2014
(51) Int. Cl.: A61B 17/10

(54) **RETAINING DEVICE**

(30) Priority: 14.01.2014 JP 2014004638
(71) Applicant: Olympus Corporation, Tokyo 192-8507 (JP)
(72) Inventor: SOBAJIMA, Hideo, Tokyo 192-8507 (JP); TAKEI, Yusuke, Tokyo 192-8507 (JP)
(74) Representative: Schicker, Silvia
(86) International application number: PCT/JP2014/077081
(87) International publication number: WO 2015/107734

(57) **Abstract**

A retaining device includes a first member, a second member, a third member, a support section supporting the first member and supporting the second member and the third member movably relative to the first member, and a manipulation part moving the first member, the second member, and the third member relative to each other. The first member and the third member can grasp a first tissue with the first tissue disposed between the first member and the second member and between the first member and the third member, and the first member and the second member can grasp the first tissue and a second tissue such that the second tissue is disposed between the first member and the second member and between the second member and the third member to contact with the first tissue with the first member and the third member grasping the first tissue.

## Description

### Technical Field

The present invention relates to a retaining device configured to retain a tissue of a living body.

Priority is claimed on Japanese Patent Application No. 2014-004638, filed January 14, 2014, the content of which is incorporated herein by reference.

### Background Art

In the related art, when a wall surface or the like of a tissue of a body is largely torn or when incision is made in an operation or the like, one side (a first tissue) and the other side (a second tissue) in the torn area or the incision area are brought together to be sutured. Here, in many cases, suture threads pass through the first tissue and the second tissue to be drawn to close these areas, and the tissues of the gap are drawn using a plurality of grasping forceps and these areas are fastened together using a clip device or a stapler device. For example, in Patent Literature 1 and Patent Literature 2, a device configured to suture a living body tissue is disclosed.

### Citation List

### Patent Literature

Patent Literature 1: Published Japanese Translation No. 2001-512703 of the PCT International Publication
Patent Literature 2: Japanese Unexamined Patent Application, First Publication No. 2010-125200

### Summary of Invention

### Technical Problem

However, in the device disclosed in Patent Literature 1, since two pairs of jaw members that can be opened and closed to grasp the tissue of the living body are provided and the two pairs can be moved relative to each other, a configuration of the device is complicated. In addition, in the device disclosed in Patent Literature 2, since a flat plate is interposed between the first tissue and the second tissue in the tissue after the suturing, the flat plate delays conglutination between the first tissue and the second tissue.

In consideration of the above-mentioned circumstances, the present invention is directed to provide a retaining device capable of easily grasping a first tissue and a second tissue of a living body such that the first tissue and the second tissue come in contact with each other, then, fastening together the first tissue and the second tissue with a tissue fastening tool in a state in which the first tissue and the second tissue are in contact with each other, and further, implanting the tissue fastening tool in a fastening area in a state in which the first tissue and the second tissue are fastened together.

### Solution to Problem

According to a first aspect of the present invention, a retaining device is configured to retain a first tissue of a living body and a second tissue of the living body spaced apart from the first tissue at a position at which the first tissue and the second tissue are adjacent to each other, and includes: a first member; a second member; a third member; a support section configured to support the first member, support the second member so as to be movable relative to the first member such that the second member is capable of approaching and moving away from the first member, and support the third member so as to be movable relative to the first member such that the third member is capable of approaching and moving away from the first member; and a manipulation part connected to the support section to move the first member, the second member, and the third member relative to each other. The first member has a first connecting section configured to connect a first piece such that the first piece in contact with the first tissue is disposed at a surface directed toward the second member in an outer surface of the first member, the first piece being included in a tissue fastening tool implanted in the first tissue and the second tissue in a state in which both of the first tissue and the second tissue are pinched by the tissue fastening tool. The second member has a second connecting section configured to connect a second piece such that the second piece in contact with the second tissue is disposed at a surface directed toward the first member in an outer surface of the second member, the second piece being included in the tissue fastening tool. The manipulation part has a switching part configured to switch a connected state in which the first piece is connected to the first connecting section and the second piece is connected to the second connecting section, and a released state in which the first piece is spaced apart from the first connecting section and the second piece is spaced apart from the second connecting section. The first member and the third member are capable of grasping the first tissue in a state in which the first tissue is disposed between the first member and the second member, and between the first member and the third member, and the first member and the second member are capable of grasping the first tissue and the second tissue such that the second tissue is disposed between the first member and the second member, and between the second member and the third member to bring the first tissue and the second tissue in contact with each other in a state in which the first member and the third member grasp the first tissue.

According to a second aspect of the present invention, in the retaining device according to the first aspect of the present invention, the support section may have: a first opening/closing section configured to support the first member and the second member such that the first member and the second member are capable of moving relative to each other such that a moving direction of the second member with respect to the first member becomes reciprocation movement in one predetermined plane; and a second opening/closing section configured to support the third member such that the third member is capable of moving relative to the first member such that an abutting direction of the third member with the first member becomes a direction along a surface crossing the one plane.

According to a third aspect of the present invention, in the retaining device according to the second aspect of the present invention, the second opening/closing section may reciprocate the third member between a position at which the third member is adjacent to the second member and a position at which the third member is adjacent to the first member.

According to a fourth aspect of the present invention, in the retaining device according to the third aspect of the present invention, the support section may include: a first support body having a rod shape and configured to support the first member and the second member; and a second support body formed in a tubular shape through which the first support body is inserted, and configured to be rotatable with respect to the first support body about a centerline of the first support body serving as a rotational center, the third member being fixed to the second support body. The second opening/closing section may reciprocate the third member between the position at which the third member is adjacent to the second member and the position at which the third member is adjacent to the first member along a circumference about the centerline of the first support body serving as a rotational center by a rotational motion of the second support body with respect to the first support body.

According to a fifth aspect of the present invention, in the retaining device according to the second aspect of the present invention, the second opening/closing section may linearly move the third member relative to the first member.

### Advantageous Effects of Invention

According to the retaining device of each of the aspects, the first tissue and the second tissue of the living body can be easily grasped such that the first tissue and the second tissue come in contact with each other, then, the first tissue and the second tissue can be fastened together by the tissue fastening tool in a state in which the first tissue and the second tissue are in contact with each other, and further, the tissue fastening tool can be implanted in a fastening area in a state in which the first tissue and the second tissue are fastened together.

### Brief Description of Drawings

Fig. 1 is a side view of a retaining device according to a first embodiment of the present invention.
Fig. 2 is a plan view showing an example of a tissue fastening tool that can be attached to the retaining device according to the first embodiment and used.
Fig. 3 is a schematic view showing a tissue fastening state using the tissue fastening tool.
Fig. 4 is a partial cross-sectional view showing a configuration of a portion of the retaining device according to the first embodiment.
Fig. 5 is a front view of the retaining device.
Fig. 6 is a front view showing a state in which the tissue fastening tool is attached to the retaining device.
Fig. 7 is a plan view of a treatment section of the retaining device.
Fig. 8 is a front view showing a state in which the tissue fastening tool is attached to the retaining device.
Fig. 9 is a view for describing an action of the retaining device.
Fig. 10 is a view for describing an action of the retaining device.
Fig. 11 is a view showing a process of a tissue fastening procedure using the retaining device.
Fig. 12 is a view showing a process of the tissue fastening procedure using the retaining device.
Fig. 13 is a view showing a process of the tissue fastening procedure using the retaining device.
Fig. 14 is a view showing a process of the tissue fastening procedure using the retaining device.
Fig. 15 is a schematic view showing a state in which the tissue fastening tool fastens a tissue.
Fig. 16 is a perspective view showing a configuration of a modified example of the first embodiment.
Fig. 17 is a partial cross-sectional view showing a retaining device according to a second embodiment of the present invention.
Fig. 18 is a front view of the retaining device according to the second embodiment.
Fig. 19 is a front view showing a configuration of a modified example of the second embodiment.
Fig. 20 is a front view showing another configuration of the modified example of the second embodiment.
Fig. 21 is a front view showing still another configuration of the modified example of the second embodiment.

### Description of Embodiments

### (First embodiment)

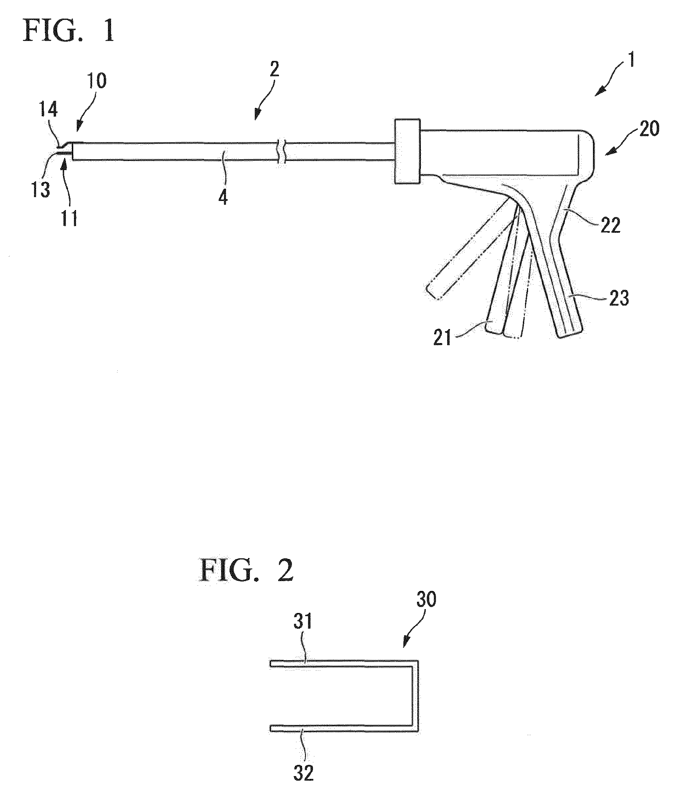

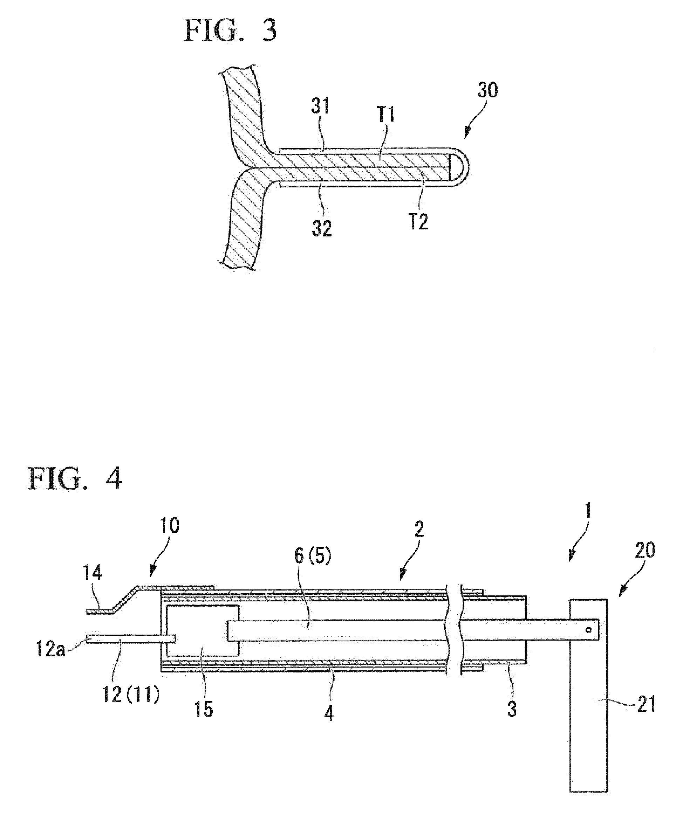

A first embodiment of the present invention will be described. Fig. 1 is a side view of a retaining device according to the embodiment. A retaining device 1 according to the embodiment shown in Fig. 1 is a device configured to retain a first tissue T1 and a second tissue T2 (see Fig. 3) of a living body in a state in which the tissues are adjacent to each other. In the embodiment, the first tissue T1 and the second tissue T2 may be different internal organs, may be two places spaced apart from each other due to an injury in an internal organ having an incision wound or a laceration, or may be two places spaced apart from each other in a normal tissue. Hereinafter, as the first tissue T1 and the second tissue T2, the two places spaced apart from each other by an incision in an operation will be exemplarily described.

First, a configuration of a tissue fastening tool 30 according to the embodiment configured to fasten together the first tissue T1 and the second tissue T2 will be described. Fig. 2 is a plan view showing an example of the tissue fastening tool that can be attached to the retaining device according to the embodiment and used. Fig. 3 is a schematic view showing a tissue fastening state using the tissue fastening tool.

The tissue fastening tool 30 shown in Fig. 2 is formed in a U shape and can be plastically deformed. The tissue fastening tool 30 is constituted by, for example, a metal. The tissue fastening tool 30 has a first piece 31 configured to come in contact with the first tissue T1 when the first tissue T1 and the second tissue T2 are fastened together, and a second piece 32 configured to come in contact with the second tissue T2 when the first tissue T1 and the second tissue T2 are fastened together. An end portion of the first piece 31 and an end portion of the second piece 32 are linked together. That is, the tissue fastening tool 30 pinches both of the first tissue T1 and the second tissue T2 as shown in Fig. 3 as the first piece 31 and the second piece 32 are closed in a state in which the first tissue T1 and the second tissue T2 are inserted between the first piece 31 and the second piece 32, and retains the first tissue T1 and the second tissue T2 in a state in which the first tissue T1 and the second tissue T2 come in contact with each other. The first tissue T1 and the second tissue T2 retained by the tissue fastening tool 30 according to the embodiment are conglutinated in the contact portion thereof.

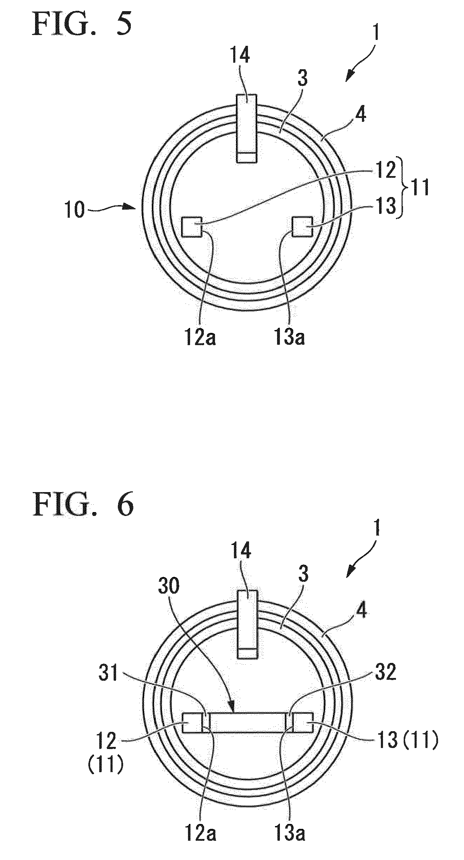

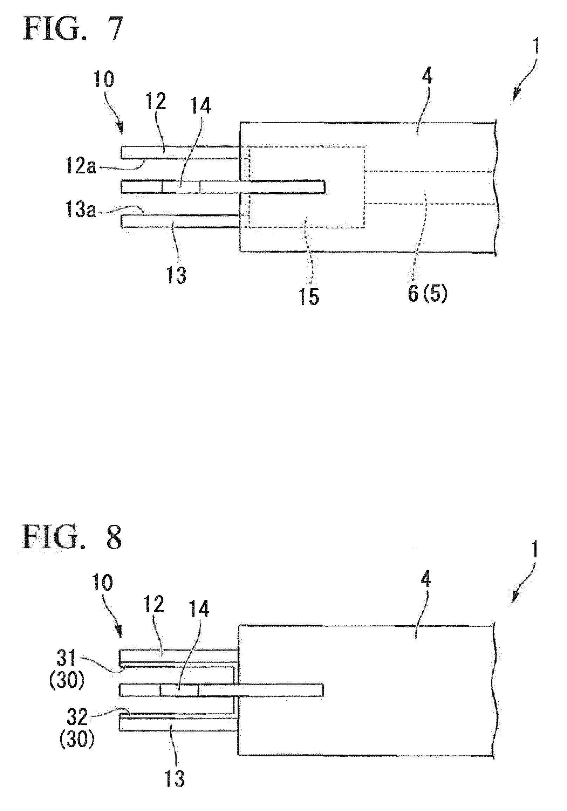

Next, a configuration of the retaining device 1 according to the embodiment will be described. Fig. 4 is a partial cross-sectional view showing a configuration of a portion of the retaining device according to the embodiment. Fig. 5 is a front view of the retaining device. Fig. 6 is a front view showing a state in which the tissue fastening tool is attached to the retaining device. Fig. 7 is a plan view of a treatment section of the retaining device. Fig. 8 is a front view showing a state in which the tissue fastening tool is attached to the retaining device.

As shown in Fig. 1, the retaining device 1 includes a rod-shaped insertion section 2 that can be inserted into a body, a treatment section 10 provided at a distal end of the insertion section 2, and a manipulation part 20 provided at a proximal end of the insertion section 2.

As shown in Fig. 4, the insertion section 2 includes an inner tube portion 3 (a first support body), an outer tube portion 4 (a second support body), and a transmission portion 5 disposed in the inner tube portion 3.

The inner tube portion 3 is an elongated rigid member. The material of the inner tube portion 3 may be, for example, a resin, a metal, or the like.

The outer tube portion 4 is an elongated rigid member having an inner diameter larger than an outer diameter of the inner tube portion 3. A material of the outer tube portion 4 may be, for example, a resin, a metal, or the like. A finger member 14 (a third member) is fixed to a distal end of the outer tube portion 4. The finger member 14 is a rod-shaped member extending parallel to a centerline of the outer tube portion 4. That is, the distal end of the outer tube portion 4 becomes a support section configured to support the finger member 14. A proximal end of the outer tube portion 4 is disposed in the vicinity of the manipulation part 20. In a state in which the inner tube portion 3 is disposed in the outer tube portion 4, the centerline of the outer tube portion 4 and a centerline of the inner tube portion 3 substantially coincide with each other. For this reason, the outer tube portion 4 is rotatable with respect to the inner tube portion 3 about the centerline of the inner tube portion 3 serving as a rotational center.

The transmission portion 5 is disposed in the inner tube portion 3 to transmit the manipulation of implanting the tissue fastening tool 30 in a living body tissue from the manipulation part 20 to the treatment section 10. The transmission portion 5 has a connecting rod 6 having a distal end fixed to a link section 15 (to be described below) in the treatment section 10 and a proximal end disposed at the manipulation part 20.

As shown in Figs. 4 and 5, the treatment section 10 includes a pair of jaws 11 (a first member 12 and a second member 13) configured to implant the tissue fastening tool 30 in the living body tissue, the above-mentioned finger member 14 (the third member) fixed to the distal end of the outer tube portion 4, and the link section 15 (a support section, a first opening/closing section) configured to support the pair of jaws 11 to be opened/closed. The pair of jaws 11 can be opened and closed by the link section 15. As shown in Figs. 6 and 8, the tissue fastening tool 30 can be attached between the pair of jaws 11.

As shown in Figs. 7 and 8, the first member 12 is formed in substantially a rod shape. A proximal end of the first member 12 is attached to the link section 15. The first member 12 has a first connecting section 12a configured to connect with the first piece 31 of the tissue fastening tool 30. The first connecting section 12a according to the embodiment is a surface directed toward the second member 13 among the outer surfaces of the first member 12.

The second member 13 is formed in substantially a rod shape. A proximal end of the second member 13 is attached to the link section 15. The second member 13 has a second connecting section 13a configured to connect with the second piece 32 of the tissue fastening tool 30. The second connecting section 13a according to the embodiment is a surface directed toward the first member 12 among the outer surfaces of the second member 13.

An interval between the first member 12 and the second member 13 defines a distance between the first piece 31 and the second piece 32 of the tissue fastening tool 30 in a state in which the first piece 31 is connected to the first connecting section 12a and the second piece 32 is connected to the second connecting section 13a (hereinafter referred to as "a connected state"). That is, when the first connecting section 12a and the second connecting section 13a move relative to each other such that the first connecting section 12a and the second connecting section 13a approach each other, the first piece 31 and the second piece 32 of the tissue fastening tool 30 approach each other, and the tissue fastening tool 30 is closed to retain the living body tissue. In addition, when the first member 12 and the second member 13 move away from each other after the tissue fastening tool 30 in the connected state is closed, as the first piece 31 moves away from the first connecting section 12a and the second piece 32 moves away from the second connecting section 13a, the tissue fastening tool 30 is released from the first member 12 and the second member 13 (hereinafter referred to as "a released state").

The link section 15 shown in Fig. 4 supports the first member 12 and the second member 13 such that the first member 12 and the second member 13 are opened and closed (see Fig. 7). The link section 15 is connected to a distal end of the connecting rod 6 of the transmission portion 5. The link section 15 converts linear movement of the connecting rod 6 into opening/closing movement between the first member 12 and the second member 13 when the connecting rod 6 moves in a centerline direction of the inner tube portion 3 in the inner tube portion 3.

The manipulation part 20 shown in Fig. 1 includes a lever 21 (see Fig. 4) connected to the proximal end of the connecting rod 6 to open/close the first member 12 and the second member 13, and a manipulation main body section 22 having a grip 23 that can be grasped by an operator together with the lever 21. That is, the manipulation part 20 is connected to the link section 15 via the connecting rod 6. The lever 21 functions as a switching part configured to switch the connected state and the released state of the tissue fastening tool 30 with respect to the first member 12 and the second member 13 by switching an opening/closing state between the first member 12 and the second member 13.

In addition, in the embodiment, the proximal end of the outer tube portion 4 is disposed in the vicinity of the boundary portion between the manipulation part 20 and the insertion section 2. The proximal portion of the outer tube portion 4 is a portion that can be manipulated by the operator to rotate the outer tube portion 4 about the centerline thereof serving as a rotational center.

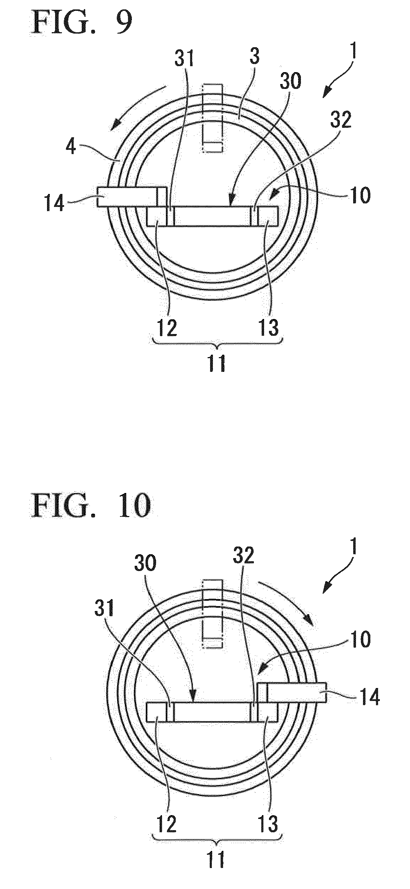

Next, an action of the retaining device 1 according to the embodiment will be described. Figs. 9 and 10 are views for describing the action of the retaining device according to the embodiment. The retaining device 1 according to the embodiment can open and close the first member 12 and the second member 13 by moving the lever 21 shown in Fig. 4. For this reason, the tissue fastening tool 30 disposed between the first member 12 and the second member 13 can be closed by moving the lever 21. In addition, the retaining device 1 according to the embodiment can rotate the finger member 14 fixed to the distal end of the outer tube portion 4 about the centerline of the inner tube portion 3 serving as a rotational center by rotating the outer tube portion 4 with respect to the inner tube portion 3 about the centerline of the inner tube portion 3 serving as a rotational center as shown in Figs. 9 and 10, independently from the opening/closing motion of the first member 12 and the second member 13.

Since the centerline of the outer tube portion 4 and the centerline of the inner tube portion 3 substantially coincide with each other, the outer tube portion 4 rotates about the centerline of the outer tube portion 4. As the outer tube portion 4 rotates about the centerline thereof serving as a rotational center, the finger member 14 rotates about the centerline of the outer tube portion 4 serving as a rotational center together with the outer tube portion 4, and abuts the first member 12. That is, the distal portion of the tube member moves the finger member 14 along a circumference about the centerline of the outer tube portion 4 and the centerline of the inner tube portion 3 concentric therewith that serve as a rotational center by rotating the outer tube portion 4 with respect to the inner tube portion 3. An abutting direction of the finger member 14 with the first member 12 is a direction along a surface crossing a plane in which the opening/closing motion of the first member 12 and the second member 13 is performed. The distal portion of the outer tube portion 4 functions as a second opening/closing section configured to open and close the finger member 14 and the first member 12.

The second opening/closing section can reciprocate the finger member 14 between a position at which the finger member 14 is close to the second member 13 and a position at which the finger member 14 is close to the first member 12. Accordingly, the operator can sandwich the tissue between the first member 12 and the finger member 14, and can sandwich the tissue between the second member 13 and the finger member 14.

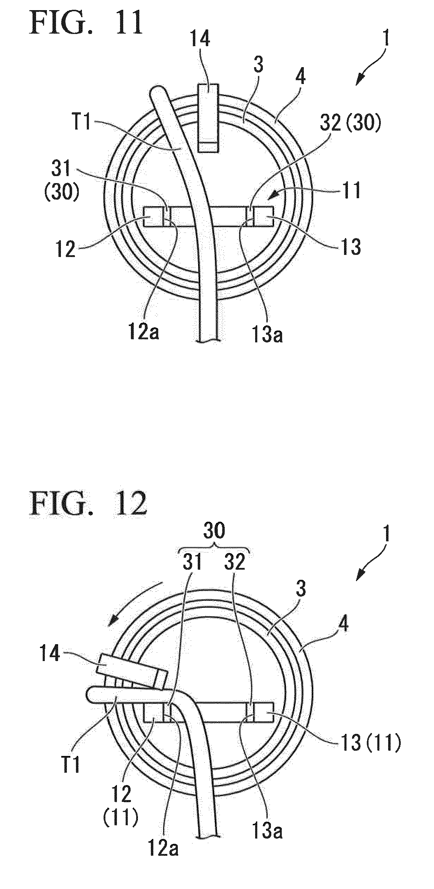

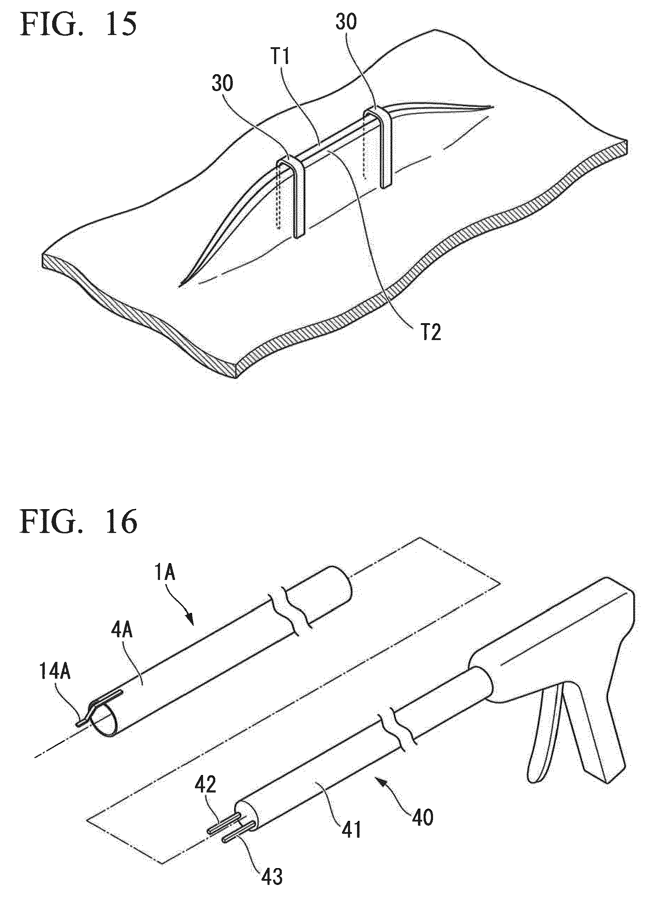

Next, an action and an effect of the retaining device 1 according to the embodiment will be described in detail by exemplarily showing a procedure using the retaining device 1 according to the embodiment. Figs. 11 to 14 are views showing a process of a tissue fastening procedure using the retaining device according to the embodiment. Fig. 15 is a schematic view showing a state in which the tissue fastening tool fastens the tissue.

In the embodiment, as the first tissue T1 and the second tissue T2 (see Fig. 3) fastened by the tissue fastening tool 30, two places formed to be spaced apart from each other by incision of the operation will be exemplarily described. Such fastening may be, for example, a procedure of closing the mesentery, closing of a stapler insertion port in intestinal anastomosis, temporary fastening in intestinal anastomosis, or the like. Hereinafter, an example of closing the mesentery incised during an operation is shown.

In use of the retaining device 1 according to the embodiment, for example, when the mesentery is closed, the insertion section 2 is inserted into the abdominal cavity through a trocar (not shown) attached to the abdominal wall. The treatment section 10 disposed at the distal end of the insertion section 2 is guided to an area to be closed by manipulation of the operator.

In the incision portion of the mesentery, first, the treatment section 10 is guided to one of the two places serving as the fastening target. Here, in the embodiment, as shown in Fig. 11, the outer tube portion 4 is positioned with respect to the inner tube portion 3 such that the finger member 14 is disposed between the first member 12 and the second member 13 or the finger member 14 comes in contact with the second member 13. Accordingly, a gap into which to insert the tissue (the first tissue T1) of the mesentery is generated between the first member 12 and the finger member 14.

The operator moves the finger member 14 with respect to the first member 12 such that the finger member 14 approaches the first member 12 by inserting the first tissue T1 between the first member 12 and the finger member 14 and rotating the outer tube portion 4 with respect to the inner tube portion 3 as shown in Fig. 12. Accordingly, the first tissue T1 is grasped by the first member 12 and the finger member 14. Here, the first tissue T1 comes in contact with the first piece 31 of the tissue fastening tool 30 connected to the first connecting section 12a of the first member 12.

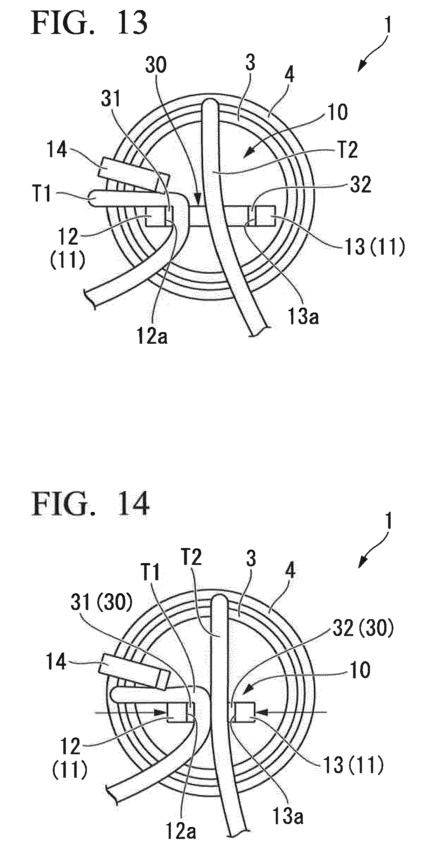

After the first tissue T1 is grasped by the treatment section 10 as described above, subsequently, the operator moves the treatment section 10 toward the other side (the second tissue T2) of the tissue serving as the closing target while maintaining a state in which the first tissue T1 is grasped between the first member 12 and the finger member 14. As the operator moves the treatment section 10, the first tissue T1 grasped by the treatment section 10 is drawn to the second tissue T2 (see Fig. 13).

After the first tissue T1 is drawn to a place at which the first tissue T1 comes in contact with the second tissue T2, the operator inserts the second tissue T2 between the first member 12 and the second member 13 as shown in Fig. 13 while maintaining a state in which the first member 12 and the finger member 14 grasp the first tissue T1. Accordingly, the second tissue T2 has a positional relation that the second tissue T2 can come in direct contact with the first tissue T1, and can come in contact with the second piece 32 of the tissue fastening tool 30 disposed at the second connecting section 13a.

The operator manipulates the lever 21 shown in Fig. 1 to close the first member 12 and the second member 13 while maintaining a state in which the first member 12 and the finger member 14 grasp the first tissue T1. The retaining device 1 retains both of the first tissue T1 and the second tissue T2 in a state in which the first tissue T1 and the second tissue T2 are brought in contact with each other by the pair of jaws 11 as shown in Fig. 14. Further, the first member 12 and the second member 13 plastically deform the tissue fastening tool 30 such that the first piece 31 and the second piece 32 of the tissue fastening tool 30 approach each other. That is, the first member 12 and the second member 13 plastically deform the tissue fastening tool 30 such that the tissue fastening tool 30 fastens together the first tissue T1 and the second tissue T2 while pinching the tissues T1 and T2.

The first tissue T1 and the second tissue T2 are retained in a state in which the first tissue T1 and the second tissue T2 are brought in direct contact with each other by the plastically deformed tissue fastening tool 30. The operator manipulates the lever 21 to open the first member 12 and the second member 13. Accordingly, the tissue fastening tool 30 is released from the first connecting section 12a of the first member 12 and the second connecting section 13a of the second member 13. Accordingly, as shown in Fig. 15, the tissue fastening tool 30 is implanted in the mesentery such that the first tissue T1 and the second tissue T2 of the mesentery serving as the closing target are closed. As necessary, a plurality of tissue fastening tools 30 may be used to fasten the tissue together.

Since the tissue fastening tool 30 fastens together the first tissue T1 and the second tissue T2 in a state in which the first tissue T1 and the second tissue T2 are in direct contact with each other, the first tissue T1 and the second tissue T2 are gradually conglutinated using a contact portion between the first tissue T1 and the second tissue T2 as a starting point. Accordingly, the incised mesentery is closed.

The procedure of closing the mesentery is performed, for example, when a portion of the intestines is excised, when a portion of the mesentery itself is excised, or when a gastric bypass operation is performed. However, the suture of the related art requires a complicated sequence to simultaneously perform a motion of drawing a portion serving as a suture target and a motion of suturing a tissue while retaining the drawn portion. In addition, for example, in an obesity operation, since the abdominal wall of a patient is thick, it may be difficult to secure a wide space in the body when the medical device including the retaining device 1 according to the embodiment is used.

In the retaining device 1 according to the embodiment, the first tissue T1 and the second tissue T2 are grasped, both of the first tissue T1 and the second tissue T2 can be retained in a state in which the first tissue T1 and the second tissue T2 are in contact with each other, then, the first tissue T1 and the second tissue T2 can be fastened together by the tissue fastening tool 30, and the tissue fastening tool 30 can be implanted in the body. For this reason, in comparison with the case in which both of the medical device configured to grasp the tissue and the clip device configured to fasten the tissue are introduced into the body, the procedure can be conveniently and rapidly preformed even in a narrow space.

In addition to the fact that the finger member 14 can reciprocate with respect to the first member 12 until the finger member 14 comes in contact with the first member 12, the finger member 14 can reciprocate with respect to the second member 13 until the finger member 14 comes in contact with the second member 13. For this reason, in the tissue serving as the closing target, even if any one of the first tissue T1 and the second tissue T2 is grasped first, the procedure can be smoothly performed until the first tissue T1 and the second tissue T2 are fastened together. Further, since the retaining device 1 according to the embodiment includes the treatment section 10 having a symmetrical structure, the tissue can be grasped and fastened together regardless of the handedness of the operator or the approach direction of the retaining device 1.

Since the second tissue T2 can be inserted between the first member 12 and the second member 13 while maintaining a state in which the first tissue T1 is grasped between the first member 12 and the finger member 14, the fastening position between the first tissue T1 and the second tissue T2 can be easily adjusted and modified.

In the embodiment, since the tissue fastening tool 30 pinches and fastens together both of the first tissue T1 and the second tissue T2 such that the first tissue T1 and the second tissue T2 come in direct contact with each other, conglutination between the first tissue T1 and the second tissue T2 is faster than when another member is disposed between the first tissue T1 and the second tissue T2.

Since the finger member 14 can be moved by rotating the outer tube portion 4 with respect to the inner tube portion 3, a manipulation force can be effectively transmitted to the finger member 14 using a simple configuration.

### (Modified example 1-1)

Next, a modified example of the first embodiment will be described. Fig. 16 is a perspective view showing a configuration of a modified example of the embodiment. As shown in Fig. 16, a retaining device 1A of the modified example is a device that can be attached to a known clip device 40. The retaining device 1A includes an outer tube portion 4A having a tubular shape through which an insertion body 41 inserted into the body in the clip device 40 can be inserted, and a finger member 14A provided at a distal end of the outer tube portion 4A. In addition, a stopper configured to define a positional relation between jaws 42 and 43 of the clip device 40 in the centerline direction of the insertion body 41 of the clip device 40 and the finger member 14A of the retaining device 1A of the modified example may be provided at the distal end of the outer tube portion 4A.

In the modified example, the retaining device 1A of the modified example is attached to the known clip device 40, and thus can perform the same procedure as the retaining device 1 described in the first embodiment. In addition, the retaining device 1A can be removed from the clip device 40 when the grasping of the tissue using the finger member 14A is not needed.

### (Second embodiment)

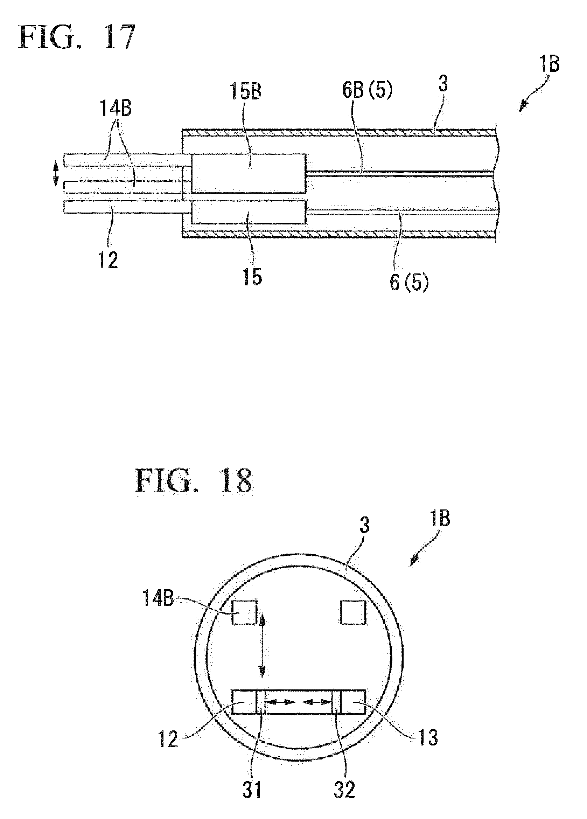

Next, a second embodiment of the present invention will be described. Fig. 17 is a partial cross-sectional view showing a retaining device according to the embodiment. Fig. 18 is a front view of the retaining device. A retaining device 1B according to the embodiment shown in Fig. 17 does not include the outer tube portion 4 and the finger member 14 that are described in the first embodiment, and instead of the outer tube portion 4 and the finger member 14, a second link section 15B and a finger member 14B are formed in the inner tube portion 3. Further, in the embodiment, unlike the first embodiment, a connecting rod 6B having a distal end connected to the second link section 15B is disposed in the inner tube portion 3. In addition, in the embodiment, unlike the first embodiment, in addition to the lever 21 configured to advance and retract the connecting rod 6 described in the first embodiment in the centerline direction of the inner tube portion 3, a manipulation unit (not shown) configured to advance and retract the connecting rod 6B according to the embodiment in the centerline direction of the inner tube portion 3 is provided at the manipulation part 20. As shown in Figs. 17 and 18, the second link section 15B converts advance and retraction motion of the connecting rod 6B into reciprocation motion of the finger member 14B to reciprocate the finger member 14B such that the finger member 14B approaches or moves away from the first member 12. In the embodiment, the second link section 15B moves the finger member 14B in a direction along a surface perpendicular to a plane on which the opening/closing motion of the first member 12 and the second member 13 is performed.

In the embodiment, in the procedure of fastening together the first tissue T1 and the second tissue T2, in the same sequence as the first embodiment, like the first embodiment, after the first tissue T1 and the second tissue T2 are drawn such that the tissues are close to each other, the tissues can be fastened together.

### (Modified example 2-1)

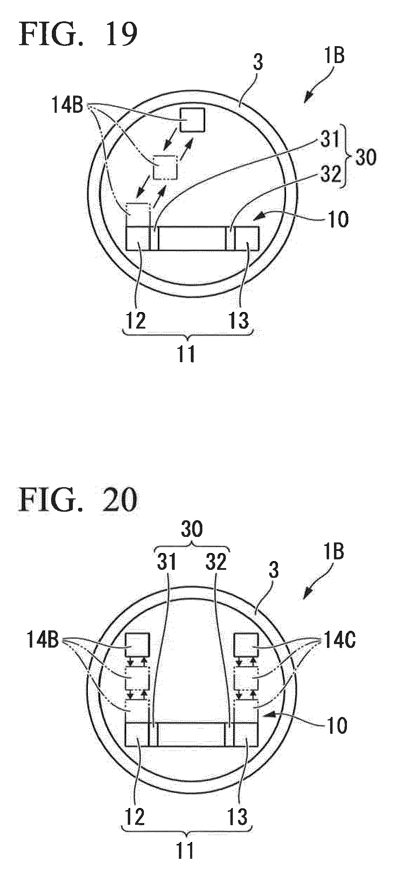

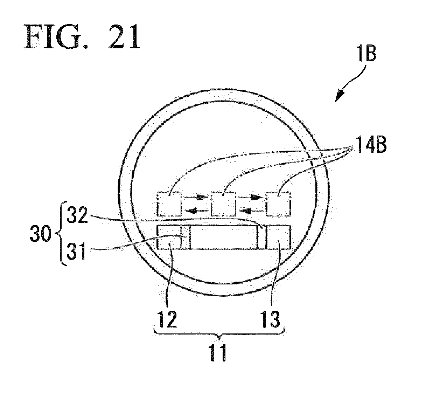

Next, a modified example of the embodiment will be described. Fig. 19 is a front view showing a configuration of the modified example of the embodiment. Fig. 20 is a front view showing another configuration of the modified example of the embodiment. Fig. 21 is a front view showing still another configuration of the modified example of the embodiment.

As shown in Fig. 19, a movement direction of the finger member 14B with respect to the first member 12 is not limited to the direction described in the above-mentioned second embodiment. For example, the second link section 15B may move the finger member 14B in a direction along a surface inclined with respect to the plane in which the opening/closing motion of the first member 12 and the second member 13 is performed. In addition, in addition to the fact that the second link section 15B approaches or moves away from the first member 12, the second link section 15B may reciprocate the finger member 14B to approach or move away from the second member 13.

As shown in Fig. 20, the retaining device 1B according to the embodiment may include, in addition to the second link section 15B, a third link section (not shown) configured to reciprocate a finger member 14C that approaches or moves away from the second member 13.

As shown in Fig. 21, the second link section 15B may move the finger member 14B in a direction along a surface parallel to a plane on which the opening/closing motion of the first member 12 and the second member 13 is performed to reciprocate the finger member 14B between a position at which the finger member 14B comes in contact with the first member 12 and a position at which the finger member 14 comes in contact with the second member 13. In this case, in a region in which the fastening of the tissue by the tissue fastening tool 30 is not disturbed, the second link section 15B supports the finger member 14B such that the finger member 14B comes in contact with the surface different from the first connecting section 12a among the outer surfaces of the first member 12 and the finger member 14B comes in contact with the surface different from the second connecting section 13a among the outer surfaces of the second member 13. Accordingly, for example, when the second tissue is inserted between the first member 12 and the second member 13 after the first tissue is grasped by the first member 12 and the finger member 14B, the first tissue and the second tissue can be fastened together while the first member 12 and the finger member 14B grasp the first tissue.

Hereinabove, while embodiments of the present invention have been described with reference to the accompanying drawings in detail, the specific configurations are not limited to the embodiments, and may include design changes or the like without departing from the scope of the present invention.

For example, as a means for fastening together a living body tissue, a clip, a stapler, or the like may be appropriately selected and employed. In addition, the inner tube portion 3, the outer tube portion 4, the transmission portion 5, and the connecting rod 6 described in the first embodiment may have flexibility. When the inner tube portion 3, the outer tube portion 4, the transmission portion 5, and the connecting rod 6 have flexibility, and the insertion section 2 is flexible as a whole, the tissue can be closed from the lumen by causing the insertion section 2 to pass through the treatment tool channel of the flexible endoscope.

The components shown in the above-mentioned embodiments and modified examples may be appropriately combined and configured.

The present invention is not limited to the above-mentioned description, and is limited only by the accompanying claims.

### Industrial Applicability

According to the retaining device of the above-mentioned embodiment, the first tissue and the second tissue of the living body can be easily grasped such that the first tissue and the second tissue come in contact with each other, then, the first tissue and the second tissue are fastened together by the tissue fastening tool in a state in which the first tissue and second tissue come in contact with each other, and further, the tissue fastening tool can be implanted in the fastening area in a state in which the first tissue and the second tissue are fastened together.

### Reference Signs List

1, 1A, 1B: retaining device
2: insertion section
3: inner tube portion
4, 4A: outer tube portion
5: transmission portion
6, 6B: connecting rod
10: treatment section
11: jaw
12: first member
12a: first connecting section
13: second member
13a: second connecting section
14, 14A, 14B: finger member (third member)
15: link section
15B: second link section
20: manipulation part
21: lever
22: manipulation main body section
23: grip
30: tissue fastening tool
31: first piece
32: second piece
40: clip device
41: insertion body
42, 43: jaw
T1: first tissue
T2: second tissue

## Claims

1. A retaining device configured to retain a first tissue of a living body and a second tissue of the living body spaced apart from the first tissue at a position at which the first tissue and the second tissue are adjacent to each other, the retaining device comprising:
a first member;
a second member;
a third member;
a support section configured to support the first member, support the second member so as to be movable relative to the first member such that the second member is capable of approaching and moving away from the first member, and support the third member so as to be movable relative to the first member such that the third member is capable of approaching and moving away from the first member; and
a manipulation part connected to the support section to move the first member, the second member, and the third member relative to each other,
wherein the first member has a first connecting section configured to connect a first piece such that the first piece in contact with the first tissue is disposed at a surface directed toward the second member in an outer surface of the first member, the first piece being included in a tissue fastening tool implanted in the first tissue and the second tissue in a state in which both of the first tissue and the second tissue are pinched by the tissue fastening tool,
the second member has a second connecting section configured to connect a second piece such that the second piece in contact with the second tissue is disposed at a surface directed toward the first member in an outer surface of the second member, the second piece being included in the tissue fastening tool,
the manipulation part has a switching part configured to switch a connected state in which the first piece is connected to the first connecting section and the second piece is connected to the second connecting section, and a released state in which the first piece is spaced apart from the first connecting section and the second piece is spaced apart from the second connecting section, and
the first member and the third member are capable of grasping the first tissue in a state in which the first tissue is disposed between the first member and the second member, and between the first member and the third member, and the first member and the second member are capable of grasping the first tissue and the second tissue such that the second tissue is disposed between the first member and the second member, and between the second member and the third member to bring the first tissue and the second tissue in contact with each other in a state in which the first member and the third member grasp the first tissue.

2. The retaining device according to claim 1, wherein the support section has:
a first opening/closing section configured to support the first member and the second member such that the first member and the second member are capable of moving relative to each other such that a moving direction of the second member with respect to the first member becomes reciprocation movement in one predetermined plane; and
a second opening/closing section configured to support the third member such that the third member is capable of moving relative to the first member such that an abutting direction of the third member with the first member becomes a direction along a surface crossing the one plane.

3. The retaining device according to claim 2, wherein the second opening/closing section reciprocates the third member between a position at which the third member is adjacent to the second member and a position at which the third member is adjacent to the first member.

4. The retaining device according to claim 3, wherein the support section includes:
a first support body having a rod shape and configured to support the first member and the second member; and
a second support body formed in a tubular shape through which the first support body is inserted, and configured to be rotatable with respect to the first support body about a centerline of the first support body serving as a rotational center, the third member being fixed to the second support body,
wherein the second opening/closing section reciprocates the third member between the position at which the third member is adjacent to the second member and the position at which the third member is adjacent to the first member along a circumference about the centerline of the first support body serving as a rotational center by a rotational motion of the second support body with respect to the first support body.

5. The retaining device according to claim 2, wherein the second opening/closing section linearly moves the third member relative to the first member.
